# EUROPÄISCHE PATENTSCHRIFT

(11) **EP 1 330 655 B1**
(45) Veröffentlichungstag und Bekanntmachung des Hinweises auf die Patenterteilung: **02.02.2005**
(21) Anmeldenummer: 01992895.1
(22) Anmeldetag: 24.10.2001
(51) Int. Cl.: G01N 33/68

(54) **VERFAHREN ZUR ANALYSE VON PROTEINEN**
METHOD FOR ANALYZING PROTEINS
PROCEDE D'ANALYSE DE PROTEINES

(30) Priorität: 31.10.2000 DE 10054055
(43) Veröffentlichungstag der Anmeldung: 30.07.2003
(73) Patentinhaber: NMI NATURWISSENSCHAFTLICHES UND MEDIZINISCHES INTITUT AN DER UNIVERSITÄT TÜBINGEN, 72770 Reutlingen (DE)
(72) Erfinder: JOOS, Thomas, 72074 Tübingen (DE); STOLL, Dieter, 72766 Reutlingen (DE)
(74) Vertreter: Otten, Hajo, Dr.-Ing.
(86) Internationale Anmeldenummer: PCT/EP2001/012295
(87) Internationale Veröffentlichungsnummer: WO 2002/037117

(56) Entgegenhaltungen:
- WO-A-00/45168
- WO-A-96/05847
- LEVILLIERS NICOLETTE ET AL: "Monoclonal and polyclonal antibodies detect a new type of post-translational modification of axonemal tubulin." JOURNAL OF CELL SCIENCE, Bd. 108, Nr. 9, 1995, Seiten 3013-3028, XP002193117 ISSN: 0021-9533
- JEAN F ET AL: "A novel protein immunoassay with predetermined specificity using monoclonal antibodies against tryptic fragments: application to HIV P24 antigen" JOURNAL OF IMMUNOLOGICAL METHODS, ELSEVIER SCIENCE PUBLISHERS B.V.,AMSTERDAM, NL, Bd. 185, Nr. 1, 11. September 1995 (1995-09-11), Seiten 103-114, XP004021158 ISSN: 0022-1759
- GENG M ET AL: "Signature-peptide approach to detecting proteins in complex mixtures" JOURNAL OF CHROMATOGRAPHY, ELSEVIER SCIENCE PUBLISHERS B.V. AMSTERDAM, NL, Bd. 870, Nr. 1-2, Februar 2000 (2000-02), Seiten 295-313, XP004187331 ISSN: 0021-9673
- GRIMM R ET AL: "Nanogram scale separations of proteins using capillary high-performance liquid chromatography with fully-automated on-line microfraction collection followed by matrix-assisted laser desorption ionisation time-of-flight mass spectrometry, protein sequencing and Western blot analysis" JOURNAL OF CHROMATOGRAPHY A, ELSEVIER SCIENCE, NL, Bd. 800, Nr. 1, 20. März 1998 (1998-03-20), Seiten 83-88, XP004112376 ISSN: 0021-9673

## Beschreibung

Die vorliegende Erfindung betrifft ein Verfahren zur Analyse von Proteinen, bei dem ein Array von für Peptidepitope spezifischen ersten Fängermolekülen eingesetzt wird.

Derartige Verfahren sind umfangreich aus dem Stand der Technik bekannt, sie dienen der qualitativen bzw. quantitativen Analyse von Proteinen.

Einsatzgebiete der bekannten Verfahren sind beispielsweise die Proteinanalytik und der Proteinnachweis. Ein weiteres, neues Anwendungsgebiet der bekannten Verfahren ist das Gebiet der Proteomik, ein Forschungszweig, der sich ganz den Proteinen widmet. Ein Aspekt der Proteomik behandelt den Vergleich der Proteinzusammensetzung in krankhaft veränderten Zellen im Gegensatz zu normalen, gesunden Zellen. Derartige Untersuchungen werden heutzutage in der Regel mittels zweidimensionaler Gel-Elektrophorese durchgeführt. Ein anderer Bereich untersucht die räumliche Struktur der Proteine, die insbesondere für Pharmaunternehmen zur Entwicklung neuer Medikamente interessant ist.

Bei der zweidimensionalen Gel-Elektrophorese wird das zu untersuchende Proteingemisch auf einen festen Träger aufgebracht, wobei eine erste Trennung nach dem Gehalt der Proteine an sauren und basischen Aminosäurebausteinen über einen pH-Gradienten erfolgt. Ein elektrisches Feld trennt anschließend die Proteine in der zweiten Richtung auf, so daß ein Fleckenmuster entsteht, in dem jeder Fleck für ein Protein steht. Durch Vergleich der Fleckenmuster können Unterschiede in der Proteinzusammensetzung zwischen gesunden und krankhaft veränderten Zellen untersucht werden. Zur Identifizierung werden die Proteinflecken ausgestanzt und die Proteine durch Verdau mit spezifischen Proteasen in Fragmente zerlegt. Die Massen der Fragmente, die nach dieser Behandlung übrig bleiben, sind für jedes Protein charakteristisch.

Bei dem eingangs erwähnten Verfahren zur Analyse von Proteinen erfolgt der Nachweis beispielsweise im Arrayformat mit proteinspezifischen Antikörpern.

Zur Gewinnung derartiger proteinspezifischer Antikörper werden bei der Anmelderin isolierte, gereinigte Proteine, rekombinant produzierte Proteine oder aus der Proteinsequenz abgeleitete, chemisch synthetisierte Peptide eingesetzt. Die Gewinnung der Antikörper erfolgt durch Immunisierung von Versuchstieren mit den als Antigenen wirkenden Proteinen bzw. Peptiden sowie mit Adjuvantien. Andererseits ist es auch möglich, die Antikörper über *in-vitro*-Verfahren als rekombinante Antikörper zu gewinnen.

Die Ableitung der chemisch zu synthetisierenden Peptide aus bekannten Proteinsequenzen, die z.B. in Proteindatenbanken enthalten sind oder aus Nukleinsäuredatenbanken ermittelt werden können, erfolgt über Software-Programme, mit denen theoretische Vorhersagen über die Proteinstruktur und eine mögliche Antigenizität getroffen werden können. Derartige Programme werden beispielsweise von Lars Hennig: "WinPep - ein Programm zur Analyse von Aminosäuresequenzen", BIOSPEKTRUM 4 (5), 1998, Seiten 49-50 oder von Devereux et al.: "A Comprehensive Set of Sequence Analysis Programs for the VAX", NUCLEIC ACIDS RESEARCH, Band 12, 1984, Seiten 387-395 beschrieben. Mit diesen Programmen lassen sich alle möglichen Fragmente mit Molekulargewicht, Sequenz, Sequenzposition und Länge aufführen, die bei einer Spaltung von Proteinen beispielsweise durch Proteasen oder chemische Agenzien erzeugt werden. Zur Vorhersage der Antigenizität wird aufgrund struktureller Vorhersagen die Wahrscheinlichkeit ermittelt, mit der das Epitop an der Proteinoberfläche liegt; siehe z.B. Hopp and Woods: "A computer program für predicting protein antigenic determinants", Mol.Immunol., Band 20, 1983, Seiten 483-489; weitere Literaturhinweise finden sich im World Wide Web unter der Adresse www.expasy.ch.

Die durch Anwendung dieser Techniken erzeugten Antikörper erkennen zwar sehr gut das zur Immunisierung eingesetzte Peptid, binden aber häufig nicht an das entsprechende Peptidepitop im nativen Protein. Dies kann beispielsweise daran liegen, daß das Peptidepitop im nativen Protein aus sterischen Gründen für den Antikörper nicht zugänglich ist oder daß es in einer infolge von posttranslationaler Modifikation modifizierten Form vorliegt, die sich aus der Datenbank nicht ableiten läßt. Ein weiterer Grund kann darin liegen, daß das Peptidepitop im nativen Protein in einer Konformation vorliegt, die eine Antikörper-Bindung verhindert.

Bei dieser Technik ist es also von Nachteil, daß zunächst aufwendig eine Vielzahl peptidspezifischer Antikörper gegen Proteinepitope erzeugt werden, die anschließend nicht oder nur schlecht an das gesuchte Protein binden.

Der Proteinnachweis mit derartigen proteinspezifischen Antikörpern kann zum einen nach Auftrennung mittels SDS-PAGE und Transfer auf Membranen über Westernblot/Immunoblot-Techniken oder mittels der ELISA-Technik erfolgen. Bei der ELISA-Technik wird das nachzuweisende Protein ohne vorherige Abtrennung anderer Proteine aus einer kompletten Proteinlösung direkt an einen hochspezifischen, an einem festen Träger immobilisierten Antikörper gebunden. Die Proteinlösung wird danach vom festen Träger abgewaschen und das gebundene, also an dem Träger verbleibende Protein nachgewiesen.

Dieser Nachweis erfolgt entweder mit Hilfe von markierten, für das gebundene Protein spezifischen Zweitantikörpern oder durch Kompetition des Analytproteins mit der Lösung in definierter Menge zugesetztem, markiertem Analytprotein. Bei diesem indirekten Nachweis wird ausgenutzt, daß unmarkiertes Analytprotein aus der Probe und zugesetztes markiertes Analytprotein in einer durch das Massenwirkungsgesetz definierten Weise um die vorhandenen Bindungsstellen konkurrieren. Daraus ergibt sich, daß die Menge des gebundenen, markierten Analytproteins umgekehrt proportional zur Menge des nicht-markierten Analytproteins in der Probe ist.

Dieses Verfahren weist zum einen den bereits erwähnten Nachteil auf, daß nämlich die Herstellung der spezifischen Antikörper zu einer Vielzahl von Antikörpern führt, die nicht oder nur schlecht an das zu untersuchende Protein binden. Beim Einsatz der Westernblot-Technik ist weiter von Nachteil, daß die durch SDS-PAGE aufgetrennte Proteinmischung jeweils nur mit einem Antikörper bzw. mit unterschiedlich markierten Antikörpern getestet werden kann, um eine Unterscheidbarkeit der an die verschiedenen Proteine gebundenen Antikörper zu gewährleisten.

Auch bei der ELISA-Technik kann immer nur ein Antikörper pro Analysenkavität immobilisiert und anschließend mit der Proteinlösung inkubiert werden. Diese Technik erfordert große Probenmengen, da jede Analysenkavität mit einem Aliquot der Proteinmischung inkubiert werden muß. Bei dem bekannten Verfahren ist weiter von Nachteil, daß der Nachweis der gebundenen Proteine spezifische Zweitantikörper für jedes nachzuweisende Protein oder große Mengen an markierten Analytproteinen für Kompetitionsexperimente erfordert.

Wie bereits eingangs erwähnt, werden bei der Anmelderin proteinspezifische Antikörper mit unterschiedlicher Spezifität auch im Arrayformat in Reihen und Spalten auf einem Trägermaterial funktionell immobilisiert. Die zu untersuchenden Proteine werden markiert und danach mit den Antikörpern auf dem Array inkubiert. Die Proteine in der Lösung, die Antigene für die immobilisierten Antikörper darstellen, binden dabei an die für sie spezifischen Antikörper, so daß sich eine ortsaufgelöste spezifische Proteinbindung ergibt.

Aufgrund der bekannten Bindungsspezifitäten der immobilisierten Antikörper und der bekannten Positionen der jeweiligen Antikörper im Array kann die gebundene Menge der jeweiligen Proteine parallel bestimmt werden. Hierzu wird mittels einer ortsaufgelösten Detektion die gebundene Proteinmenge über die Markierung an den Proteinen gemessen.

Neben dem qualitativen, parallelen Nachweis verschiedener in der Probenlösung vorhandener Proteine kann durch Einsatz von Standardproteinen zusätzlich eine quantitative Bestimmung der Analytproteine erfolgen.

Im Gegensatz zu dem oben beschriebenen Verfahren ist hier von Vorteil, daß in derselben Probe und in einer Kavität mehrere Proteine parallel detektiert werden können. Von Nachteil ist jedoch, daß die zu analysierenden Proteine markiert werden müssen, wozu entsprechende Markierungen an bestimmten funktionellen Gruppen einzelner Aminosäuren der Proteine eingeführt werden. Die Effizienz derartiger Markierungsreaktionen ist bei verschiedenen Proteinen sehr unterschiedlich, sie wird durch die jeweilige direkte Mikroumgebung einer funktionellen Gruppe bestimmt, also durch sterische Abschirmung, pH-Variation in direkter Umgebung der funktionellen Gruppe durch Nachbargruppen, Salze, Solventien usw. Vom Ergebnis her bedeutet dies, daß die Reaktivität chemisch identischer funktioneller Gruppen in einem Protein sehr unterschiedlich sein kann, so daß eine quantitative Umsetzung bestimmter funktioneller Gruppen sehr schwierig ist.

Darüber hinaus kann die Markierungsreaktion dazu führen, daß Aminosäuren innerhalb eines vom spezifischen Antikörper erkannten Epitops modifiziert werden, was zum Verlust der Bindung zwischen Proteinepitop und Antikörper führt. Damit ist aber das Protein nach der Markierung durch das beschriebene Testverfahren nicht mehr nachweisbar.

In der Veröffentlichung Levilliers et al.: "Monoclonal and polyclonal antibodies detect a new type of post-translational modification of axonemal tubulin", JOURNAL OF CELL SCIENCE, Band 108, Nr. 9, 1995, Seiten 3013-3028, wird die Analyse von α- und β-Tubulin beschrieben, wobei die Proteine abgebaut und mit Antikörpern inkubiert werden. Die Identität und die Position der Antikörper im Gel ist nicht bekannt.

Vor diesem Hintergrund liegt der vorliegenden Erfindung die Aufgabe zugrunde, das eingangs genannte Verfahren derart weiterzubilden, daß eine quantitative und/oder qualitative Analyse von Proteinen auf einfache, schnelle und zuverlässige weise möglich wird.

Erfindungsgemäß wird diese Aufgabe durch folgendes Verfahren gelöst:
Verfahren zum qualitativen und/oder quantitativen Nachweis von Analytproteinen in einer Proteinmischung, mit den Schritten:
Bereitstellen von in einem Array in bekannter Anordnung immobilisierten ersten Fängermolekülen, an die spezifisch bekannte Peptidepitope der Analytproteine binden,
Abbau von in der Proteinmischung enthaltenen Proteinen zu Peptidfragmenten, die den Peptidepitopen entsprechen, die für die Generierung der ersten Fängermoleküle eingesetzt wurden, wenn die Analytproteine in der Proteinmischung enthalten sind,
Inkubation des Arrays mit den Peptidfragmenten, und
Nachweis von an die ersten Fängermoleküle gebundenen Peptidfragmenten.

Die der Erfindung zugrunde liegende Aufgabe wird auf diese Weise vollkommen gelöst. Der Erfindung liegt die überraschende Erkenntnis der Erfinder zugrunde, daß die im Arrayformat immobilisierten Fängermoleküle nicht wie im Stand der Technik mit den Proteinen sondern nach beispielsweise enzymatischer Spaltung der Proteine mit einzelnen Peptidfragmenten inkubiert werden, die den Peptidepitopen entsprechen, die für die Generierung der spezifischen ersten Fängermoleküle eingesetzt werden.

Bei diesem Verfahren ist zum einen von Vorteil, daß die Sekundär- und Tertiärstruktur der zu analysierenden Proteine keine Rolle spielt, die ersten Fängermoleküle erkennen die entsprechenden Peptidfragmente mit großer Sicherheit, da sie gegen entsprechende Peptidepitope generiert wurden. Diese Epitope können unmittelbar aus Datenbanken vorhergesagt werden, eine zusätzliche aufwendige Vorhersage von Sekundär- und Tertiärstruktur ist nicht erforderlich. Auf diese Weise ist das neue Verfahren nicht nur sehr zuverlässig, es ist auch einfach und schnell durchzuführen, da die ersten Fängermoleküle zuverlässig die Proteine über die nachgewiesenen Peptidfragmente erkennen.

Ein weiterer Vorteil ist darin zu sehen, daß jedes Fängermolekül sein optimales Antigen erkennt, denn das synthetische Peptid, das zur Erzeugung von Fängermolekülen verwendet wird, ist identisch zu dem nachzuweisenden Peptidfragment. Auf diese Weise wird der im Stand der Technik vorhandene Nachteil vermieden, daß bei Proteinen nämlich nicht vorhersagbar ist, ob ein peptidspezifischer Antikörper auch das native Protein erkennt. Mit anderen Worten bedeutet dies, daß der Ausschuß bei den ersten Fängermolekülen deutlich gegenüber dem Stand der Technik verringert ist.

Da die Anordnung der unterschiedlichen ersten Fängermoleküle in dem Array bekannt ist, kann mittels einer automatisierbaren, ortsaufgelösten Detektion eine qualitative Identifizierung und quantitative Bestimmung der Fragmente und damit der Proteine erfolgen. Die gefundenen Peptidmuster korrelieren nämlich direkt mit dem Proteinmuster, das bei dem Entwurf des Arrays als Analytprotein festgelegt wurde. Außerdem können gefundene Peptidmuster mit einfachen, schnellen Algorithmen gegen DNA-Datenbanken gescreent werden.

Auf diese Weise ist eine schnelle und zuverlässige Aussage darüber möglich, welche Proteine in einer Probenlösung vorhanden sind, was beispielsweise bei der Diagnostik eingesetzt werden kann. Ferner ist mit dem neuen Verfahren erstmals eine einfache Mutationsanalytik auf Proteinebene möglich.

Ein weiterer Vorteil bei dem bekannten Verfahren liegt in seiner Geschwindigkeit, denn die niedermolekularen Fragmente erlauben die schnellere Durchführung von Bindungsassays als hochmolekulare Proteine. Es ergibt sich somit eine kinetische Beschleunigung der Assays, wobei eine ähnliche Kinetik der Analytbindung zur einfacheren Etablierung optimaler Bedingungen für die Assays führt, als dies bei der stark variierenden Kinetik der Fall ist, die sich für in ihrem Molekulargewicht stark variable Analytproteine ergibt.

Ein weiterer Vorteil des neuen Verfahrens besteht darin, daß der Abbau der zu analysierenden Proteine zu den Peptidfragmenten definiert und vollständig erfolgen kann, und daß sich die Fragmente im Gegensatz zu Proteinen an definierten funktionellen Gruppen quantitativ markieren lassen. Dies führt dazu, daß der Nachweis der Peptidfragmente nicht nur qualitativ sondern auch quantitativ möglich wird, ohne daß auf Sekundärund Tertiärstrukturen geachtet werden muß.

In einer Weiterbildung ist es dann bevorzugt, wenn nach der Inkubation ungebundene Peptidfragmente weggewaschen werden.

Hier ist von Vorteil, daß in an sich bekannter Weise die Spezifität des Verfahrens erhöht wird.

Weiter ist es bevorzugt, wenn die Peptidfragmente nach der Inkubation markiert werden.

Da eine spezifische, vollständige Markierung der Peptidfragmente an definierten funktionellen Gruppen möglich ist, läßt sich auf diese Weise eine quantitative Bestimmung jedes gebundenen Peptidepitopes durchführen. Dabei ist von Vorteil, daß eine vollständige Markierung der Peptidfragmente leichter und reproduzierbarer möglich ist als eine Markierung von Proteinen, wie dies im Stand der Technik durchgeführt wird.

Weiter ist es bevorzugt, wenn der Nachweis über markierte zweite Fängermoleküle erfolgt, die spezifisch das an das erste Fängermolekül gebundene Peptidfragment erkennen.

Hier ist zum einen von Vorteil, daß die Peptidfragmente nicht markiert werden müssen, so daß das Verfahren insgesamt schneller durchgeführt werden kann, denn markierte zweite Fängermoleküle können in einem einzigen Verfahrensschritt in großer Menge zur Verfügung gestellt werden, woraufhin dann viele erfindungsgemäße Analyseverfahren mit unterschiedlichen Proben von Proteinen durchgeführt werden können, die jeweils nur zu den entsprechenden Peptidfragmenten abgebaut werden müssen.

Ein weiterer Vorteil dieser Maßnahme besteht darin, daß die Selektivität erhöht wird, denn unspezifisch vom ersten Fängermolekül gebundene Peptidfragmente werden nicht erkannt, da die zweiten Fängermoleküle an diese Komplexe nicht binden.

Dabei ist es bevorzugt, wenn die zweiten Fängermoleküle ausgehend von Komplexen aus ersten Fängermolekülen und daran gebundenen Peptidepitopen erzeugt werden.

Hier ist von Vorteil, daß sehr spezifische zweite Fängermoleküle erzeugt werden, die zudem mit hoher Erfolgsquote herstellbar sind.

Insgesamt ist es bevorzugt, wenn die ersten Fängermoleküle ausgehend von Peptidepitopen erzeugt werden, die gezielt im Hinblick auf Peptidfragmente hergestellt werden, die bei dem Abbau der zu analysierenden Proteine entstehen.

Diese Maßnahme ist mit einer ganzen Reihe von Vorteilen verbunden. Zum einen lassen sich die ersten Fängermoleküle gegen die linearen Peptidepitope mit hoher Erfolgsquote produzieren, da die Peptidepitope zur Herstellung bzw. Isolierung der Fängermoleküle mit den zu analysierenden Peptidepitopen vollständig identisch sind. Dies führt dazu, daß bei der Herstellung der ersten Fängermoleküle erheblich weniger Ausschuß produziert wird, als bei der Herstellung von proteinspezifischen Fängermolekülen. Solche Peptidepitope zur Herstellung bzw. Isolierung der Fängermoleküle können schnell und preiswert hergestellt und vollständig analytisch charakterisiert werden, was einen weiteren Vorteil gegenüber dem Stand der Technik darstellt.

Darüber hinaus wird durch diese Maßnahme auch die Spezifität des neuen Verfahrens erhöht. In verschiedenen Proteinen vorkommende Epitope können nämlich durch weitere proteinspezifische Epitope so ergänzt werden, daß die Kombination der auf dem Array nachgewiesenen Epitope für bestimmte Analytproteine eindeutig ist. Auf diese Weise werden nicht nur Spezifität und Redundanz des neuen Verfahrens erhöht, es ist auch eine gezielte Optimierung für den Nachweis bestimmter Proteine möglich.

Die Peptidepitope können dabei durch chemische Synthese oder enzymatischen Abbau aus bekannten Proteinen hergestellt werden, so daß sie mit Standardmethoden kostengünstig und schnell vollständig analytisch charakterisierbar sind.

Dabei ist es weiter bevorzugt, wenn die gezielt hergestellten Peptidepitope ausgewählt werden aus potentiellen Peptidepitopen der zu analysierenden Proteine.

Hier ist zum einen von Vorteil, daß nur Peptidepitope hergestellt werden, gegen die sich auch Fängermoleküle erzeugen lassen. Diese Maßnahme führt also zu einer Zeit- und Syntheseersparnis bei der Herstellung der ersten und/oder zweiten Fängermoleküle. Unter "potentiellen" Peptidepitopen werden dabei alle möglichen Epitope eines Proteins verstanden, also auch solche, die nicht auf der Oberfläche des Proteins vorliegen und bisher nicht für die Generierung von Fängermolekülen verwendet wurden. Dabei ist weiter von vorteil, daß pro Protein viel mehr mögliche Epitope in Betracht gezogen werden können als im Stand der Technik, so daß z.B. bei sehr ähnlichen oder nahe verwandten Proteinen eine viel größere Auswahl an Peptidepitopen zur möglichen Unterscheidung zwischen den Proteinen zur Verfügung steht.

Darüber hinaus ist von Vorteil, daß nur solche Peptidepitope hergestellt werden, die auch für ein Protein bzw. für wenige Proteine spezifisch sind.

Dabei ist es bevorzugt, wenn die potentiellen Peptidepitope aus bekannten Aminosäure- und/oder Nukleinsäuresequenzen der zu analysierenden Proteine abgeleitet werden.

Hier ist zum einen von Vorteil, daß die Vorhersage der Peptidepitope von nachzuweisenden Proteinen aus DNA- oder Proteindatenbanken ohne Berücksichtigung der Proteintertiärstruktur erfolgt. Eine einfache Vorhersage der Proteinspaltstellen für die zur spezifischen Proteinspaltung eingesetzten Agenzien reicht aus, wobei eine Auswahl von einem oder mehreren Peptidepitopen mit möglichst hoher Spezifität für das Analytprotein erfolgt. Im Optimalfall tritt das Epitop nur im Analytprotein auf.

Weiter ist es bevorzugt, wenn als potentielle Peptidepitope solche Sequenzabschnitte ausgewählt werden, die bei dem Abbau der zu analysierenden Proteine erhalten bleiben.

Bei dieser Maßnahme ist die Zeitersparnis von Vorteil, es werden nämlich nur solche Fängermoleküle erzeugt, für die auch Peptidfragmente entstehen können, wobei bei den potentiellen Peptidepitopen darauf geachtet wird, daß sie für eines oder für wenige Analytproteine spezifisch sind und daß ihre Herstellung mit hoher Wahrscheinlichkeit möglich ist.

Die Vorhersage der bei der chemischen Synthese schwierig zugänglichen Peptidsequenzen kann als weiteres Kriterium zur Auswahl der Peptidepitope herangezogen werden. Die klassische Vorhersage immunogener linearer Peptidsequenzen, bei der aufgrund der Hydropathieprofile (Kyte and Doolittle, "A Simple Method for Displaying the Hydropathic Character of a Protein", J. MOL. BIOL 157 (1982), Seiten 105-132) und der Vorhersage von β-Loopinduzierenden Sequenzabschnitten (Chou and Fasman, "Prediction of Protein Conformation", BIOCHEMISTRY 13, 1974, Seiten 222-245) potentielle lineare Peptidepitope in Proteinstrukturen identifiziert werden, ist hier nicht erforderlich, da nach Denaturierung und Abbau der Analytproteine alle Peptidfragmente unabhängig von ihrer Position in der Proteinstruktur potentielle Peptidepitope darstellen.

Dies führt zu einer größeren Auswahl an potentiellen Peptidepitopen und damit zu einer größeren Wahrscheinlichkeit, für zu analysierende Proteine (im folgenden: Analytproteine) spezifische Epitope zu finden, als bei Arrays aus Fängermolekülen, die mit vollständigen Analytproteinen inkubiert werden.

Weiter ist es bevorzugt, wenn die gezielt hergestellten Peptidepitope so markiert und/oder modifiziert werden, wie die Peptidfragmente nach/bei dem Abbau der zu analysierenden Proteine.

Bei dieser Maßnahme ist zum einen von Vorteil, daß sie zu einer hohen Spezifität der ersten Fängermoleküle gegen die Peptidfragmente führt, da bereits bei der Herstellung der zur Erzeugung der ersten Fängermoleküle verwendeten Peptidepitope berücksichtigt wird, ob die Peptidfragmente nach/bei dem Abbau der zu analysierenden Proteine markiert bzw. modifiziert werden.

Ein weiterer Vorteil ist in dem gezielten Einbau von Markierungen bei automatischer chemischer Synthese zu sehen, wodurch eine schnelle und preiswerte, vollständig definierte Markierung möglich wird, was einfacher, preiswerter und reproduzierbarer ist als eine Markierung von Gesamtprotein.

Dabei ist es bevorzugt, wenn die gezielt hergestellten Peptidepitope mit posttranslationalen Modifikationen versehen werden.

Bei dieser Maßnahme ist von Vorteil, daß die zu analysierenden Proteine gezielt auf posttranslationale Modifizierungen hin gescreent werden können. Dabei können beispielsweise Peptidepitope aus dem Bereich potentieller Phosporylierungsstellen eines Proteins als synthetische Phosphorpeptide und als unphosphorylierte Peptide hergestellt werden, um Fängermoleküle gegen die jeweiligen Peptidepitope zu generieren. Ein Array aus derartigen Fängermolekülen, die die unterschiedlichen phosphorylierten bzw. unphosphorylierten Peptidepitope erkennen, kann dann eingesetzt werden, um Phosphorylierungszustände eines oder mehrerer Proteine direkt aus einer Proteinmischung heraus zu quantifizieren. Andere Modifikationen, die auf diese Weise gescreent werden können, umfassen modifizierte Aminosäuren, Glykopeptide etc.

Weiter ist es bevorzugt, wenn die gezielt hergestellten Peptidepitope gegenüber den potentiellen Peptidepitopen zumindest einen Aminosäureaustausch und/oder zumindest eine Deletion einer Aminosäure aufweisen.

Bei dieser Maßnahme ist von Vorteil, daß ein Single Nucleotide Polymorphismus erkannt werden kann, denn der Austausch bzw. die Deletion einzelner Aminosäuren in Proteinen kann auf diese Weise detektiert und quantifiziert werden. Dazu werden Epitope aus dem Bereich des Aminosäureaustausches als synthetische Peptide hergestellt, um spezifische Fängermoleküle gegen diese Epitope zu generieren. Nach enzymatischem Abbau der entsprechenden Proteine und ggf. Markierung der generierten Peptidfragmente sowie Inkubation mit dem Array können die durch den Single Nucleotide Polymorphismus möglichen verschiedenen Peptide mit den unterschiedlichen Aminosäureaustauschen und -deletionen direkt auf dem Array nachgewiesen und quantifiziert werden.

Insgesamt ist es bevorzugt, wenn das oder jedes Fängermolekül ein Antikörper, ein Antikörperfragment, ein Peptid-Aptamer oder sonstiges durch chemische Synthese oder Mutation von Bindungsdomänen rekombinant herstellbares Fängermolekül ist.

Weiter ist es bevorzugt, wenn die Bindungsspezifität der Fängermoleküle durch Bindungstests mit in einzelnen Aminosäuren oder in ihrer Länge variierten Peptiden ermittelt wird. Dadurch ist eine vollständige Charakterisierung der Spezifität der generierten Fängermoleküle zur Vermeidung von Kreuzreaktivität gegen andere Epitope schnell und kostengünstig möglich. Bei Proteinen selbst ist dies mit vertretbarem Aufwand nicht durchführbar.

Dies kann erfindungsgemäß nun entweder im Array mit synthetischen Peptiden jeweils einer definierten Sequenz oder mit Peptidbibliotheken gegen einzelne, immobilisierte Fängermoleküle und anschließender Identifizierung gebundener Peptide erfolgen.

Das Grundprinzip des Verfahrens zur quantitativen und/oder qualitativen Analyse von Proteinen besteht darin, ein Array von für Peptidepitope spezifischen ersten Fängermolekülen einzusetzen, wobei nicht Proteine sondern nach beispielsweise enzymatischer Spaltung der Proteine einzelne Peptidfragmente, die den Peptidepitopen entsprechen, mit den im Arrayformat immobilisierten proteinspezifischen Fängermolekülen nachgewiesen werden.

Dazu werden zunächst potentielle Peptidepitope oder Proteinsequenzen in Protein- oder Gensequenzdatenbanken theoretisch vorhergesagt. Da bei der enzymatischen oder chemischen Spaltung der nachzuweisenden Proteine einzelne Epitope geteilt werden können, muß zur Vorhersage potentieller Peptidepitope die Schnittspezifität der zum Proteinabbau verwendeten Proteasen berücksichtigt werden. Potentielle Peptidepitope, die nach diesen Kriterien für die einzelnen Proteine theoretisch vorhergesagt wurden, werden mittels chemischer Peptidsynthese hergestellt und an spezifischen funktionellen Gruppen, beispielsweise der Aminofunktion des N-Terminus oder der ε-Aminogruppe von Lysin, beispielsweise mit Fluorophor, Biotin etc. markiert.

Diese Peptidepitope werden dann zur an sich bekannten Herstellung von Antikörpern durch Immunisierung verwendet. Die so hergestellten Fängermoleküle, die im einfachen Fall Antikörper sein können, werden dann in Reihen und Spalten auf dem Array immobilisiert.

Durch enzymatische oder chemische Abbaureaktion aller Proteine der gesamten zu analysierenden Proteinmischung mit spezifischen Proteasen, wie beispielsweise Trypsin, Endoprotease Lys C etc., werden den Peptidepitopen entsprechende Proteinfragmente gewonnen, die dann mit dem Array inkubiert und anhand ihrer Bindung an die ersten Fängermoleküle nachgewiesen werden.

Zum Nachweis können zum einen die erhaltenen Peptidfragmente mit einem Marker, wie beispielsweise Fluorophor, Biotin etc., an denselben spezifischen funktionellen Gruppen markiert werden, wie die zur Erzeugung der spezifischen Fängermoleküle eingesetzten synthetischen Peptidepitope. Die entsprechend markierten Peptide werden auf den Antikörperarrays inkubiert, die nicht gebundenen Peptidfragmente weggewaschen und die gebundenen Peptidfragmente über ihre Markierung ortsspezifisch nachgewiesen. Da eine vollständige Markierung der Peptide möglich ist, kann eine quantitative Bestimmung eines jeden gebundenen Peptidepitopes erfolgen.

Andererseits können auch nicht-markierte Peptidepitope nachgewiesen werden, wenn ein markiertes zweites Fängermolekül eingesetzt wird, das im einfachsten Falle ein Zweitantikörper sein kann, der das am immobilisierten Antikörper gebundene Peptid erkennt. Dieser Zweitantikörper wird gegen das am immobilisierten Antikörper gebundene Peptid generiert, wodurch es zu einer drastischen Erhöhung der Selektivität kommt, da nur das spezifische Peptidfragment am immobilisierten ersten Antikörper erkannt wird und dort unspezifisch gebundene Peptidfragmente vom zweiten Antikörper nicht gebunden werden.

Auf diese Weise werden nur Peptide, die potentielle Immunogene darstellen und beim Proteinabbau nicht fragmentiert werden, für die Immunisierung eingesetzt, was zu einer Ersparung bei der chemischen Peptidsynthese und der Herstellung spezifischer Fängermoleküle führt, da hochaffine, peptidfragmentspezifische Fängermoleküle mit hoher Erfolgsquote produziert werden können. Dies führt zu weniger Fängermolekülausschuß als bei der Herstellung proteinspezifischer Fängermoleküle.

Die Bindungsspezifität der gewonnenen Fängermoleküle, hier also Antikörper im Arrayformat, kann durch Bindungstest mit in einzelnen Aminosäuren variierten definierten synthetischen Peptiden erfolgen. Alternativ können auch synthetische Peptidbibliotheken, die alle theoretisch möglichen Peptidsequenzen enthalten, mit einzelnen, z.B. auf Affinitätschromatographiesäulen immobilisierten Antikörpern inkubiert werden. Die aus den Peptidgemischen gebundenen Peptide können nach Denaturierung des Antikörpers eluiert und massenspektrometrisch oder durch Edman-Abbau identifiziert werden.

Mit dem neuen Verfahren können Proteine aus dem Abbau von Zellen, aus Körperflüssigkeiten oder Geweben nicht nur quantifiziert sondern beispielsweise auch auf posttranslationale Modifikationen sowie Single Nucleotide Polymorphismen hin untersucht werden.

## Patentansprüche

1. Verfahren zum qualitativen und/oder quantitativen Nachweis von Analytproteinen in einer Proteinmischung, mit den Schritten:
Bereitstellen von in einem Array in bekannter Anordnung immobilisierten ersten Fängermolekülen, an die spezifisch bekannte Peptidepitope der Analytproteine binden,
Abbau von in der Proteinmischung enthaltenen Proteinen zu Peptidfragmenten, die den Peptidepitopen entsprechenden, die für die Generierung der ersten Fängermoleküle eingesetzt wurden, wenn die Analytproteine in der Proteinmischung enthalten sind,
Inkubation des Arrays mit den Peptidfragmenten, und
Nachweis von an die ersten Fängermoleküle gebundenen Peptidfragmenten.

2. Verfahren nach Anspruch 1, **dadurch gekennzeichnet, daß** nach der Inkubation ungebundene Peptidfragmente weggewaschen werden.

3. Verfahren nach Anspruch 1 oder 2, **dadurch gekennzeichnet, daß** die Peptidfragmente vor der Inkubation markiert werden.

4. Verfahren nach einem der Ansprüche 1 bis 3, **dadurch gekennzeichnet, daß** der Nachweis über markierte zweite Fängermoleküle erfolgt, die spezifisch das an das erste Fängermolekül gebundene Peptidfragment erkennen.

5. Verfahren nach Anspruch 4, **dadurch gekennzeichnet, daß** die zweiten Fängermoleküle ausgehend von Komplexen aus ersten Fängermolekülen und daran gebundenen Peptidepitopen erzeugt werden.

6. Verfahren nach einem der Ansprüche 1 bis 5, **dadurch gekennzeichnet, daß** die ersten Fängermoleküle ausgehend von Peptidepitopen erzeugt werden, die gezielt im Hinblick auf Peptidfragmente hergestellt werden, die bei dem Abbau der Analytproteine entstehen.

7. Verfahren nach Anspruch 6, **dadurch gekennzeichnet, daß** die gezielt hergestellten Peptidepitope ausgewählt werden aus potentiellen Peptidepitopen der Analytproteine.

8. Verfahren nach Anspruch 7, **dadurch gekennzeichnet, daß** die potentiellen Peptidepitope aus bekannten Aminosäure- und/oder Nukleinsäuresequenzen der Analytproteine abgeleitet werden.

9. Verfahren nach Anspruch 7 oder 8, **dadurch gekennzeichnet, daß** als potentielle Peptidepitope solche Sequenzabschnitte ausgewählt werden, die bei dem Abbau der Analytproteine erhalten bleiben.

10. Verfahren nach einem der Ansprüche 6 bis 9, **dadurch gekennzeichnet, daß** die gezielt hergestellten Peptidepitope so markiert und/oder modifiziert werden, wie die Peptidfragmente nach/bei dem Abbau der Analytproteine.

11. verfahren nach einem der Ansprüche 6 bis 10, **dadurch gekennzeichnet, daß** die gezielt hergestellten Peptidepitope mit posttranslationalen Modifikationen versehen werden.

12. Verfahren nach einem der Ansprüche 6 bis 11, **dadurch gekennzeichnet, daß** die gezielt hergestellten Peptidepitope gegenüber den potentiellen Peptidepitopen zumindest einen Aminosäureaustausch und/oder eine Deletion einer Aminosäure aufweisen.

13. Verfahren nach einem der Ansprüche 1 bis 12, **dadurch gekennzeichnet, daß** das oder jedes Fängermolekül ein Antikörper, ein Antikörperfragment, ein Peptid-Aptamer oder ein sonstiges, durch chemische Synthese oder Mutation von Bindungsdomänen rekombinant herstellbares Fängermolekül ist.

14. Verfahren nach einem der Ansprüche 1 bis 13, **dadurch gekennzeichnet, daß** die Bindungsspezifität der Fängermoleküle durch Bindungstests mit in einzelnen Aminosäuren oder ihrer Länge variierten Peptiden ermittelt wird.

## Claims

1. A method for qualitatively and/or quantitatively detecting analyte proteins in a protein mixture, comprising the steps of:
providing of first capture molecules that are immobilized in known arrangement in an array, known peptide epitopes specifically binding to said first capture molecules,
in case said analyte proteins are present in said protein mixture, degradation of proteins present in said protein mixture into peptide fragments that correspond to said peptide epitopes used for the generation of said first capture molecules,
incubation of the array with the peptide fragments, and
detection of peptide fragments bound to the first capture molecules.

2. The method of claim 1, **characterized in that** unbound peptide fragments are washed away after said incubation.

3. The method of claim 1 or 2, **characterized in that** the peptide fragments are labeled before said incubation.

4. The method of anyone of claims 1 to 3, **characterized in that** the detection takes place via labeled second capture molecules which specifically recognize the peptide fragment bound to the first capture molecule.

5. The method of claim 4, **characterized in that** the second capture molecules are generated starting from complexes of first capture molecules and peptide epitopes bound thereto.

6. The method of anyone of claims 1 to 5, **characterized in that** the first capture molecules are generated starting from peptide epitopes which are prepared in purposeful manner in relation to peptide fragments resulted from degradation of the analyte proteins.

7. The method of claim 6, **characterized in that** the purposeful prepared peptide epitopes are selected from potential peptide epitopes of the analyte proteins.

8. The method of claim 7, **characterized in that** the potential peptide epitopes are derived from known amino acid and/or nucleic acid sequences of the analyte proteins.

9. The method of claim 7 or 8, **characterized in that** sequence sections which are retained on degradation of the proteins to be analyzed are selected as potential peptide epitopes.

10. The method of anyone of claims 6 to 9, **characterized in that** the purposeful prepared peptide epitopes are labeled and/or modified like the peptide fragments after/during the degradation of the analyte proteins.

11. The method of anyone of claims 6 to 10, **characterized in that** the purposeful prepared peptide epitopes are provided with post-translational modifications.

12. The method of anyone of claims 6 to 11, **characterized in that** compared with the potential peptide epitopes the purposeful prepared peptide epitopes have at least one amino acid exchange and/or one deletion of an amino acid.

13. The method of anyone of claims 1 to 12, **characterized in that** the or each capture molecule is an antibody, an antibody fragment, a peptide aptamer or another capture molecule which can be prepared recombinantly by mutation of binding domains or by chemical synthesis.

14. The method of anyone of claims 1 to 13, **characterized in that** the binding specificity of the capture molecules is determined by binding assays using peptides which have been varied in individual amino acids or in their length.

## Revendications

1. Procédé pour la détection qualitative et/ou quantitative de protéines d'analyte dans un mélange de protéines comprenant les étapes consistant en :
la préparation de premières molécules de liaison immobilisées dans un array dans une disposition connue, molécules auxquelles se lient les épitopes peptidiques spécifiquement connus des protéines d'analyte,
la décomposition des protéines contenues dans le mélange de protéines en fragments peptidiques, qui correspondent aux épitopes peptidiques qui ont été utilisés pour la génération des premières molécules de liaison lorsque les protéines d'analyte sont contenues dans le mélange de protéines,
l'incubation de l'array avec les fragments peptidiques, et
la détection des fragments peptidiques fixés aux premières molécules de liaison.

2. Procédé selon la revendication 1, **caractérisé en ce que** des fragments peptidiques non liés sont éliminés par lavage après l'incubation.

3. Procédé selon la revendication 1 ou 2, **caractérisé en ce que** les fragments peptidiques sont marqués avant l'incubation.

4. Procédé selon l'une des revendications 1 à 3, **caractérisé en ce que** la détection s'effectue par des secondes molécules de liaison marquées qui reconnaissent spécifiquement le fragment peptidique fixé à la première molécule de liaison.

5. Procédé selon la revendication 4, **caractérisé en ce que** les secondes molécules de liaison sont produites à partir de complexes issus des premières molécules de liaison et des épitopes peptidiques fixés à celles-ci.

6. Procédé selon l'une des revendications 1 à 5, **caractérisé en ce que** les premières molécules de liaison sont produites à partir d'épitopes peptidiques, qui sont fabriqués de manière ciblée compte tenu des fragments peptidiques, qui se forment lors de la décomposition des protéines d'analyte.

7. Procédé selon la revendication 6, **caractérisé en ce que** les épitopes peptidiques fabriqués de manière ciblée sont choisis parmi des épitopes peptidiques potentiels des protéines d'analyte.

8. Procédé selon la revendication 7, **caractérisé en ce que** les épitopes peptidiques potentiels sont dérivés des séquences connues d'acide aminé et/ou d'acide nucléique des protéines d'analyte.

9. Procédé selon la revendication 7 ou 8, **caractérisé en ce que** comme épitopes peptidiques potentiels, on choisit des segments de séquences qui restent conservées lors de la décomposition des protéines d'analyte.

10. Procédé selon l'une des revendications 6 à 9, **caractérisé en ce que** les épitopes peptidiques fabriqués de manière ciblée sont marqués et/ou modifiés de la même manière que les fragments peptidiques après/lors de la décomposition des protéines d'analyte.

11. Procédé selon l'une des revendications 6 à 10, **caractérisé en ce que** les épitopes peptidiques fabriqués de manière ciblée sont pourvus de modifications post-translationnelles.

12. Procédé selon l'une des revendications 6 à 11, **caractérisé en ce que**, comparés aux épitopes peptidiques potentiels, les épitopes peptidiques fabriqués de manière ciblée comportent au moins une substitution d'acide aminé et/ou une délétion d'un acide aminé.

13. Procédé selon l'une des revendications 1 à 12, **caractérisé en ce que** la ou chaque molécule de liaison est un anticorps, un fragment d'anticorps, un aptamère de peptide ou une autre molécule de liaison pouvant être produite de manière recombinante par synthèse chimique ou mutation de domaines de liaison.

14. Procédé selon l'une des revendications 1 à 13, **caractérisé en ce que** la spécificité de liaison des molécules de liaison est déterminée par des tests de liaison avec des peptides modifiés dans chaque acide aminé ou dans leur longueur.
